# EUROPEAN PATENT APPLICATION

(11) **EP 3 686 120 A1**
(43) Date of publication of application: **29.07.2020**
(21) Application number: 20152967.4
(22) Date of filing: 21.01.2020
(51) Int. Cl.: B65D 21/02

(54) **INTERLOCKING SINGLE USE TATTOO INK CONTAINER**

(30) Priority: 28.01.2019 US 201916259375
(71) Applicant: Intenze Products, Inc., Hackensack, NJ 07601 (US)
(72) Inventor: Barth, Mario, Washington Township, NJ New Jersey NJ 07676 (US)
(74) Representative: Greaves Brewster LLP

(57) **Abstract**

The container includes an ink well and a base for the ink well. The ink well has a side defining an open top. It also has a bottom surface. The base includes a base wall configured to receive the well. The base wall has an upper section surrounding and engaging the well side proximate the open top of the well. It also has a lower section extending downwardly from the upper section. The lower base wall section includes at least one set of spaced legs. The spaced legs define a recess adapted to receive a leg of another similarly shaped container in order to interlock the containers in various arrangements.

## Description

### TECHNICAL FIELD

The present invention relates to containers for tattoo ink and more particularly to a single use, pre-poured, tattoo ink container designed to adhere to a work surface to minimize spills and being knocked over while dipping, and which is configured to interlock with other similar containers to further increase stability when multiple containers are used.

### BACKGROUND

Humans have marked their bodies with tattoos for thousands of years. These permanent designs-sometimes plain, sometimes elaborate, always personal-have served as amulets, status symbols, declarations of love, signs of religious beliefs, adornments and even forms of punishment.

The earliest known examples of tattoos on bodies were for a long time believed to be Egyptian and were present on several female mummies dated to c. 2000 B.C. But following the more recent discovery of the Iceman from the area of the Italian-Austrian border in 1991 and his tattoo patterns, this date has been pushed back a further thousand years when he was carbon-dated at around 5,200 years old.

Tattooing has been practiced in many countries in the world and has attained varying popularity throughout the centuries. Currently, tattooing is making a strong comeback. It is more popular and accepted than it has ever been. All classes of people seek the best tattoo artists. This rise in popularity has placed individuals that create tattoos in the category of "fine artist". Many artists combine the tradition of tattooing with their personal style creating unique and phenomenal body art. With the addition of new inks, tattooing has reached a new plateau.

Tattoo inks of various types have been and are used to create tattoos. The tattoo inks have been provided in a variety of different containers. However, issues of contamination have resulted from the use of ink from a single source for multiple tattoo recipients. Those issues have been addressed through the use single use ink containers.

Because only small quantities of a particular color are generally required for each individual being tattooed, single use tattoo inks can be supplied in small containers. However, the use of small single use ink containers creates other problems. Small containers tend to be difficult to fill. They are inherently unstable, and easily spilled or knock over while dipping, resulting in a messy work surface.

Those issues are addressed by the container of the present invention, which is a single use, pre-poured, tattoo ink container designed to adhered to a work surface to minimize spills and being knocked over while dipping, and which is configured to interlock with other similarly shaped containers in a variety of arrangements for enhanced stability.

### SUMMARY OF INVENTION

In accordance with a first aspect of the present invention, a single use container is provided for tattoo ink. The container includes an ink well and a base for the ink well. The ink well has a side defining an open top. It also has a bottom surface. The base includes a base wall configured to receive the well. The base wall has an upper portion surrounding and engaging the well side proximate the open top of the well. It has a lower section extending downwardly from the section which includes at least one set of spaced legs. The spaced legs define recess adapted to receive a leg of another similarly shaped container.

The container has a pad attached to said bottom surface. The pad has an exposed surface. A layer of adhesive is situated on the exposed pad surface. The adhesive layer allows the container to adhere to a work surface. A removable cover is provided to protect the adhesive layer prior to use.

The container has a removeable lid. The adapted to cover the open top of the well.

The lower section of the container has a surface. Each of the legs includes a body radially extending from the lower section surface. Each of the legs also includes a substantially circumferentially directed part extending from the body at a location spaced from the lower section surface. Preferably, first and second substantially circumferentially directed parts extend in opposite directions from each leg the body, at a location spaced from the lower section surface.

The exterior surface of the leg body is arcuate. Preferably, the exterior surface of the leg body is concave.

Preferably, the lower base wall section includes a second set of spaced legs. The the second set of spaced legs defines a recess adapted to receive a leg of another similarly shaped container.

In accordance with another aspect of the present invention, a first container for tattoo ink and a second container for tattoo ink are provided. Each of the first and second containers includes an ink well and a base for the ink well. The ink well has a side defining an open top. The ink well also has a bottom surface. In each container the base includes a wall adapted to receive the well. The base wall includes an upper portion surrounding and engaging the well side proximate the open top of well. It also includes a lower section extending downwardly from the upper section. The lower base wall section includes at least one set of spaced legs defining a recess therebetween. The recess of the first container is shaped and sized to receive a leg of the second container.

The container also includes an adhesive layer on the bottom surface of the well designed to adhere the container to a work surface. A removable cover for the adhesive layer is provided for protecting the adhesive layer prior to use of the container.

The container further includes a removeable lid. The lid is adapted to cover the open top of the well.

The lower section of the base wall has a surface. Each of the legs includes a body radially extending from the lower section surface. It also includes a substantially circumferentially directed part extending from the body at a location spaced the lower section surface. Preferably, the body includes first and second substantially circumferentially directed parts extending in opposite directions from the body, at a location spaced from the lower section surface.

The exterior surface of the leg body is arcuate. Preferably, the exterior surface of the leg body is concave.

The lower base wall section of the first container has a second set of spaced legs.

The second set of spaced legs of the first container defines a recess adapted to receive a leg of the second container.

### BRIEF DESCRIPTION OF DRAWINGS

To these and to such other objects that may hereinafter appear, the present invention relates to an interlocking single use tattoo ink container as described in detail in the following specification and recited in the annexed claims, taken together with the accompanying drawings in which:

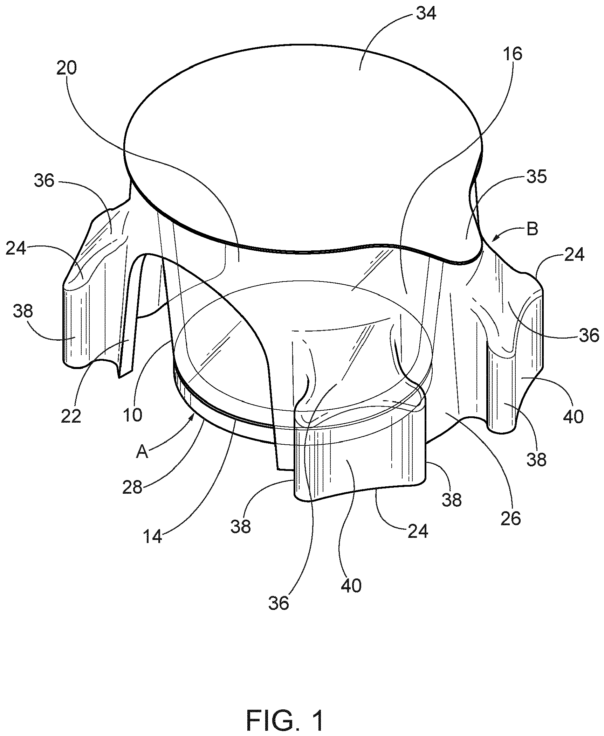

Figure 1 is a perspective view of the container of the present invention;

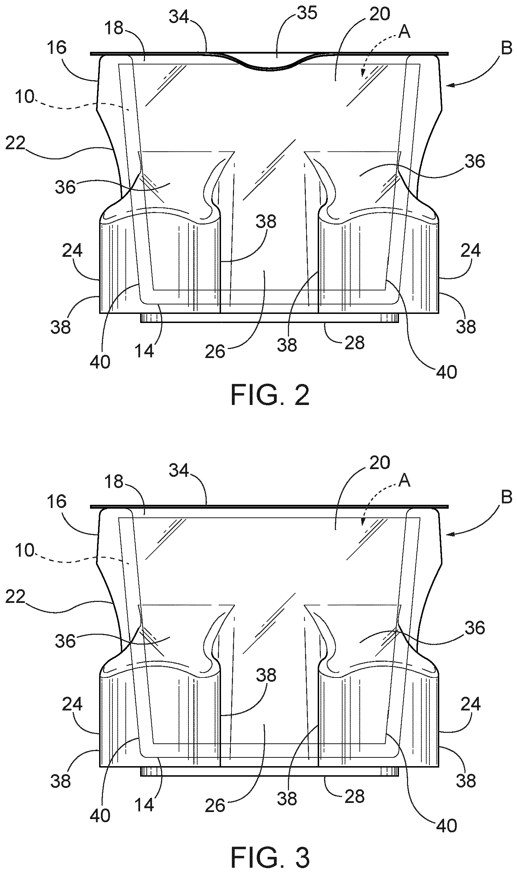

Figure 2 is a front elevation view of the container;
Figure 3 is a rear elevation view of the container;

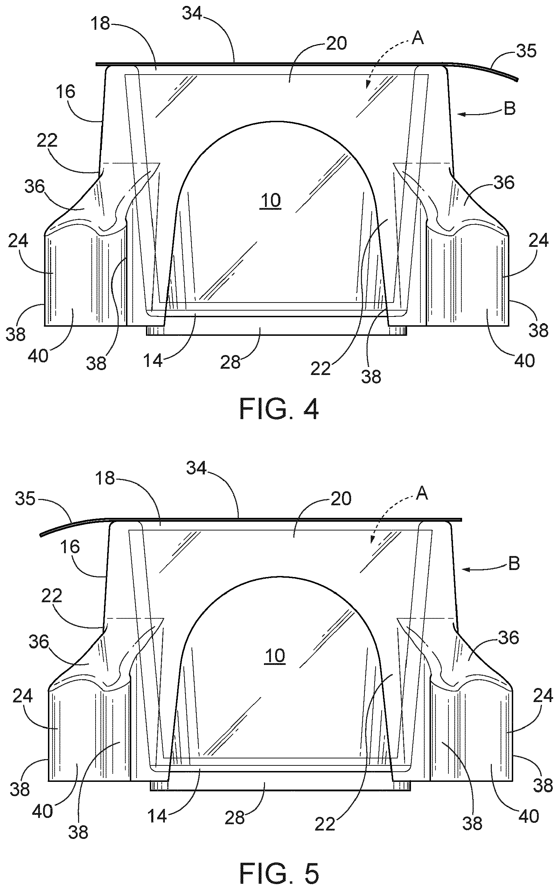

Figure 4 is an elevation view of the container showing the left side thereof;
Figure 5 is an elevation view of the container showing the right side thereof;

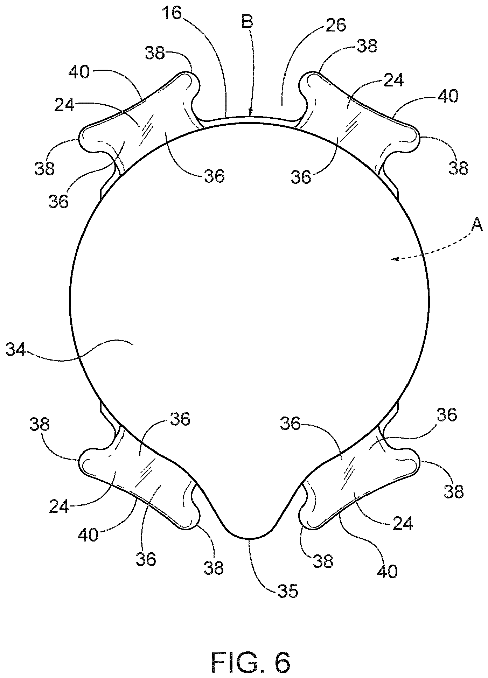

Figure 6 is a top plan view of the container;

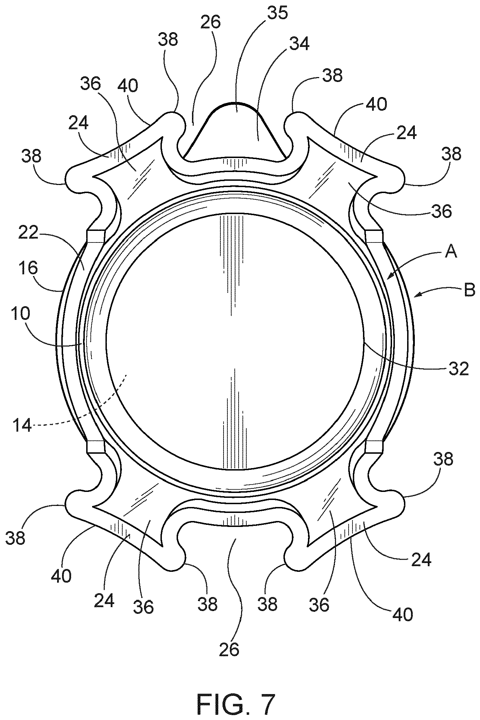

Figure 7 is a bottom plan view of the container;

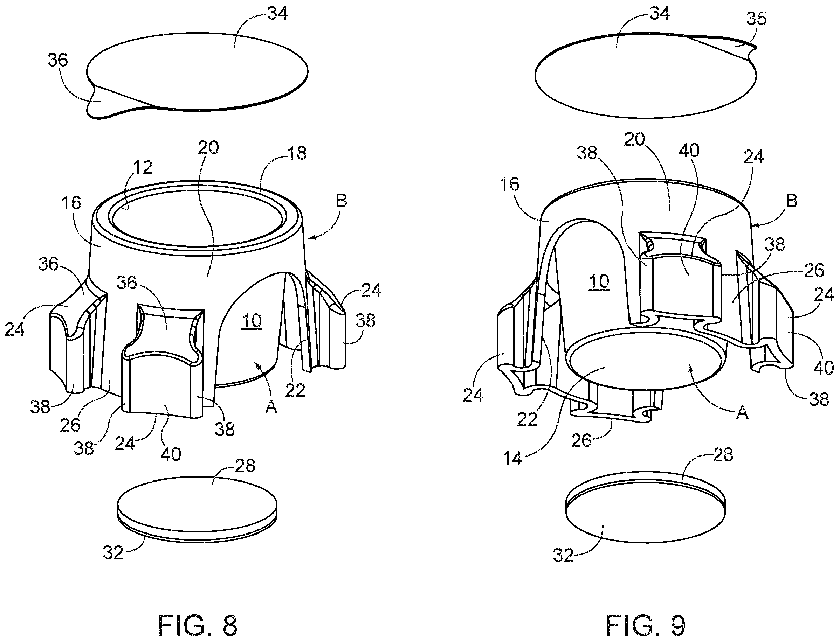

Figure 8 is an exploded perspective view of the container showing the top, front and one side thereof;
Figure 9 is an exploded perspective view of the container showing the bottom, rear and other side thereof;
Figure 10 is a perspective view of the container showing the removeable adhesive cover;
Figure 11 is a perspective view of the container showing the removeable lid;
Figure 12 shows a single container as it would appear in use;
Figure 13 shows a series of interlocked containers connected in a generally linear arrangement; and
Figure 14 shows a series of interlocked containers connected in a generally circular arrangement.

### DESCRIPTION OF EMBODIMENTS

As seen in the drawings, the tattoo ink container of the present invention includes an ink well, generally designated A, and a base for the ink well, generally designated B. Ink well A has a generally cylindrical, slightly tapered side wall 10. Side wall 10 defines an open top 12 of the well. The well also has a circular bottom surface 14.

Base B includes a wall 16 defining an opening 18 configured to receive well A in the top of the base. Base wall 16 includes a top section 20 surrounding and engaging the side of the well, proximate opening 18.

The base wall has a bottom section 22 extending downwardly from the top section 20. Bottom base wall section 22 includes at least one set of legs 24. The legs in each set are spaced from each other along base wall section 22. Preferably, two sets of legs 24 are provided on each the container, located on opposite sides thereof. The distance between the leg sets is greater than the distance between the legs in each set. A generally arcuate opening in the lower base wall section is situated between each of the leg sets.

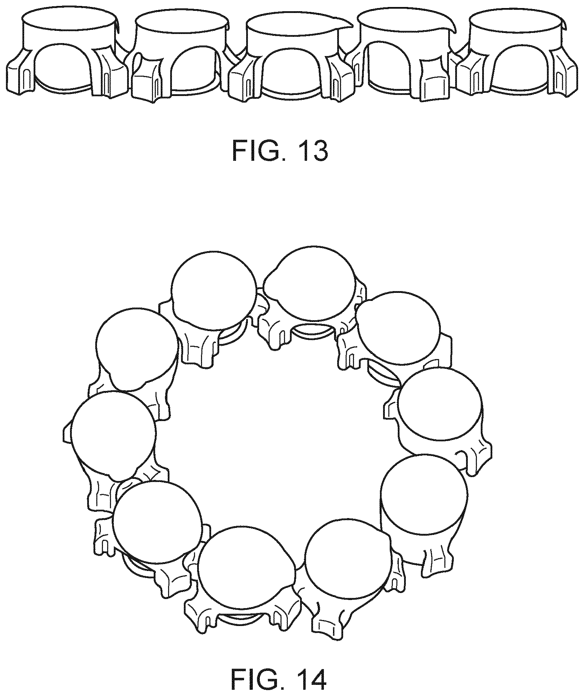

Legs 24 are preferably hollow and define a recess 26 between the legs. Recess 26 is shaped and sized to receive a leg of another similarly shaped container such that the containers can be interlocked. This allows the containers to be arranged on a work surface and connected to in various configurations to enhance the stability of the containers, as depicted in Figures 13 and 14.

The container has a felt-like circular pad 28 which is attached to the bottom surface 14 of the well. The bottom (exposed) surface of pad 28 is coated with a layer of adhesive 30. A removable protective cover 32 is situated over the adhesive layer 30 to protect the adhesive layer prior to use of the container.

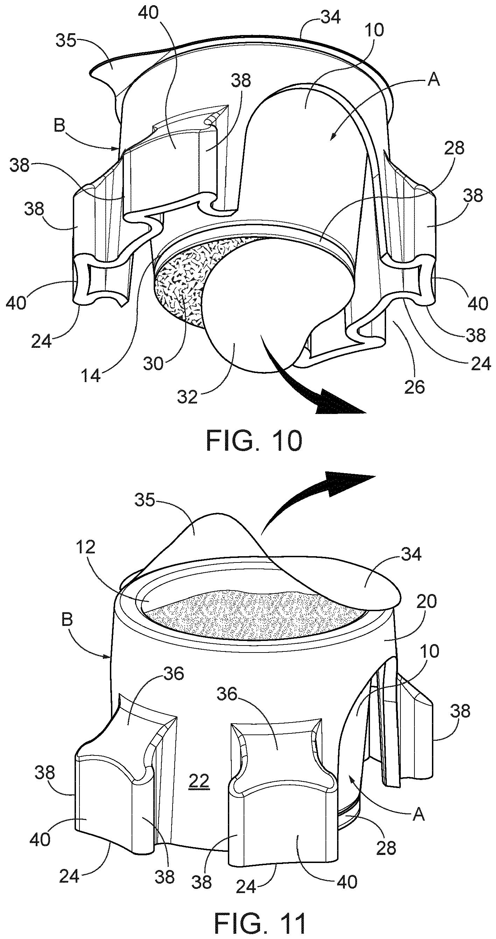

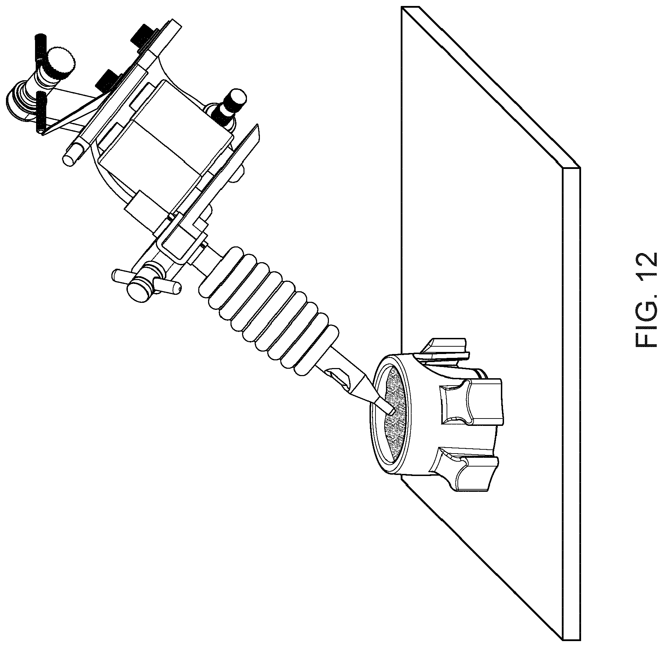

In use, cover 32 is removed from the bottom surface of the pad 28 to expose the adhesive layer 30, as seen in Figure 10. That allows the bottom of the container, and particularly the surface of pad 28, to adhere to the work surface, as seen in Figure 12, preventing the container from being accidently spilled or knocked over by the tattoo tool.

The container also has a removeable lid 34 which protects and isolates the ink in the well from the environment. Lid 34 is situated over the top opening 18 of the well after the ink is loaded into the well. Lid 34 keeps the ink in the well free from contamination until it is ready to be used. Lid 34 has a pull tab 35 to facilitate the removal of the lid.

Each of the legs 26 has a body 36 extending radially from the surface of the bottom section 22 of the base wall. First and second substantially circumferentially directed parts 38 extend in substantially opposite directions from the leg body 36 at a location spaced from the surface of the bottom section of the base wall.

The exterior surface 40 of each leg 26 is arcuate. Preferably, the surface is concave.

Figures 13 and 14 illustrate how the containers may be interlocked. Figure 13 shows a series of interlocked containers arranged in a generally linear configuration. Figure 14 shows a series of interlocked containers arranged in a generally circular linear configuration.

While only a single preferred embodiment of the present invention has been disclosed for purposes of illustration, it is obvious that many modifications and variations could be made thereto. It is intended to cover all of those modifications and variations which fall within the scope of the present invention, as defined by the following claims.

### Clauses

The following clauses define particular aspects and embodiments of the invention.
Clause 1. A single use container for tattoo ink comprising an ink well and a base for said well, said well comprising a side defining an open top, and a bottom surface, said base comprising a wall configured to receive said well, said base wall comprising a first section surrounding and engaging said well side proximate said open top thereof and a second section extending downwardly from said first section, said second base wall section comprising at least one set of spaced legs defining a recess adapted to receive a leg of another similarly shaped container.
Clause 2. The container of clause 1 further comprising a pad attached to said bottom surface, said pad having an exposed surface, and a layer of adhesive on said exposed pad surface.
Clause 3. The container of clause 2 further comprising a removable protective cover for said adhesive layer.
Clause 4. The container of clause 1 further comprising a removeable lid adapted to cover said open top of said well.
Clause 5. The container of clause 1 wherein said second section has a surface, each of said legs comprises a body radially extending from said second section surface and a substantially circumferentially directed part extending from said leg body at a location spaced from said second section surface.
Clause 6. The container of clause 1 wherein said second section has a surface, each of said legs comprises a body radially extending from said second section surface and first and second substantially circumferentially directed parts extending in opposite directions from said leg body, at a location spaced from said second section surface.
Clause 7. The container of clause 5 wherein said leg body has an exterior surface and wherein said exterior surface of said leg body is arcuate.
Clause 8. The container of clause 7 wherein said arcuate surface is concave.
Clause 9. The container of clause 1 wherein said second base wall section comprises a second set of spaced legs.
Clause 10. The container of clause 9 wherein said second set of spaced legs defines a recess adapted to receive a leg of another similarly shaped container.
Clause 11. A first single use container for tattoo ink and a second single use container for tattoo ink, each of said first and second containers comprising an ink well and a base for said well, said well comprising a side defining an open top, and a bottom surface, said base comprising a wall adapted to receive said well, said base wall comprising a first portion surrounding and engaging said well side proximate said open top thereof and a second section extending downwardly from said first section, said second base wall section comprising at least one set of spaced legs defining a recess therebetween, wherein the recess of said first container is shaped and sized to receive a leg of said second container.
Clause 12. The container of clause 11 further comprising a pad fixed to said bottom surface, said pad having a bottom surface and an adhesive layer on said bottom surface of said pad.
Clause 13. The container of clause 12 further comprising a removable protective cover for said adhesive layer.
Clause 14. The container of clause 11 further comprising a removeable lid adapted to cover said open top of said well.
Clause 15. The container of clause 11 wherein said second section has a surface, each of said legs comprises a body radially extending from said second section surface and a substantially circumferentially directed part extending from said body at a location spaced from said second section surface.
Clause 16. The container of clause 11 wherein said second section has a surface, each of said legs comprises a body radially extending from said second section surface and first and second substantially circumferentially directed parts extending in opposite directions from said body, at a location spaced from said second section surface.
Clause 17. The container of clause 15 wherein the surface of said leg body is arcuate.
Clause 18. The container of clause 17 wherein said arcuate surface is concave.
Clause 19. The container of clause 11 wherein said second base wall section of said first container comprises a second set of spaced legs.
Clause 20. The container of clause 19 wherein said second set of spaced legs of said first container defines a recess adapted to receive a leg of said second.

## Claims

1. A single use container for tattoo ink comprising an ink well and a base for said well, said well comprising a side defining an open top, and a bottom surface, said base comprising a wall configured to receive said well, said base wall comprising a first section surrounding and engaging said well side proximate said open top thereof and a second section extending downwardly from said first section, said second base wall section comprising at least one set of spaced legs defining a recess adapted to receive a leg of another similarly shaped container.

2. The container according to claim 1, further comprising a pad attached to said bottom surface, said pad having an exposed surface, and a layer of adhesive on said exposed pad surface, and optionally further comprising a removable protective cover for said adhesive layer.

3. The container according to claim 1 or claim 2, further comprising a removeable lid adapted to cover said open top of said well.

4. The container according to any preceding claim, wherein said second section has a surface, each of said legs comprises a body radially extending from said second section surface and a substantially circumferentially directed part extending from said leg body at a location spaced from said second section surface.

5. The container according to any of claims 1 to 3, wherein said second section has a surface, each of said legs comprises a body radially extending from said second section surface and first and second substantially circumferentially directed parts extending in opposite directions from said leg body, at a location spaced from said second section surface.

6. The container according to claim 4 or claim 5, wherein said leg body has an exterior surface and wherein said exterior surface of said leg body is arcuate, and optionally wherein said arcuate surface is concave.

7. The container according to any preceding claim, wherein said second base wall section comprises a second set of spaced legs.

8. The container according to claim 7, wherein said second set of spaced legs defines a recess adapted to receive a leg of another similarly shaped container.

9. A first single use container for tattoo ink and a second single use container for tattoo ink both according to claim 1, wherein the recess of said first container is shaped and sized to receive a leg of said second container.

10. The containers according to claim 9, further comprising a pad fixed to said bottom surface, said pad having a bottom surface and an adhesive layer on said bottom surface of said pad, and optionally, further comprising a removable protective cover for said adhesive layer.

11. The containers according to claim 9 or claim 10, further comprising a removeable lid adapted to cover said open top of said well.

12. The containers according to any of claims 9 to 11, wherein said second section has a surface, each of said legs comprises a body radially extending from said second section surface and a substantially circumferentially directed part extending from said body at a location spaced from said second section surface.

13. The containers according to any of claims 9 to 11, wherein said second section has a surface, each of said legs comprises a body radially extending from said second section surface and first and second substantially circumferentially directed parts extending in opposite directions from said body, at a location spaced from said second section surface.

14. The containers according to claim 12 or claim 13, wherein the surface of said leg body is arcuate, and optionally wherein said arcuate surface is concave.

15. The containers according to any of claims 9 to 14, wherein said second base wall section of said first container comprises a second set of spaced legs, and optionally wherein said second set of spaced legs of said first container defines a recess adapted to receive a leg of said second container.
